(19) Europäisches Patentamt
European Patent Office
Office européen des brevets

(11) **EP 2 928 009 A1**

(12) **EUROPEAN PATENT APPLICATION**
published in accordance with Art. 153(4) EPC

(43) Date of publication:
**07.10.2015 Bulletin 2015/41**

(21) Application number: **13858313.3**

(22) Date of filing: **28.11.2013**

(51) Int Cl.:
***H01M 14/00*** *(2006.01)* ***C09B 23/00*** *(2006.01)*
***H01B 1/12*** *(2006.01)* ***H01L 31/04*** *(2014.01)*
***H01M 2/08*** *(2006.01)*

(86) International application number:
**PCT/JP2013/082004**

(87) International publication number:
**WO 2014/084296 (05.06.2014 Gazette 2014/23)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **30.11.2012 JP 2012262251**

(71) Applicant: **Nippon Kayaku Kabushiki Kaisha**
**Tokyo 100-0005 (JP)**

(72) Inventors:
- **SATAKE Masamitsu**
  **Tokyo 115-8588 (JP)**
- **HOSHI Takayuki**
  **Tokyo 115-8588 (JP)**
- **SHIGAKI Koichiro**
  **Tokyo 115-8588 (JP)**

(74) Representative: **Gille Hrabal**
**Brucknerstrasse 20**
**40593 Düsseldorf (DE)**

(54) **DYE-SENSITIZED SOLAR CELL**

(57) The purpose of the present invention is to provide a dye-sensitized solar cell which has excellent photoelectric conversion efficiency and excellent durability. Disclosed is a dye-sensitized solar cell which comprises: a first conductive supporting body which has a semiconductor-containing layer that is sensitized by a dye; a second conductive supporting body which has a counter electrode and is disposed at a position where the semiconductor-containing layer and the counter electrode face each other at a predetermined distance; a charge transfer layer which is sandwiched between the first and second conductive supporting bodies; and a sealing agent which is provided in the peripheral portions of the first and second conductive supporting bodies for the purpose of sealing the charge transfer layer. The dye is an organic non-ruthenium dye; the counter electrode contains platinum; and the charge transfer layer is composed of an electrolyte solution that contains iodine, iodine ions and a compound which has both a thioester bond and a positively charged nitrogen atom in each molecule.

FIG.1

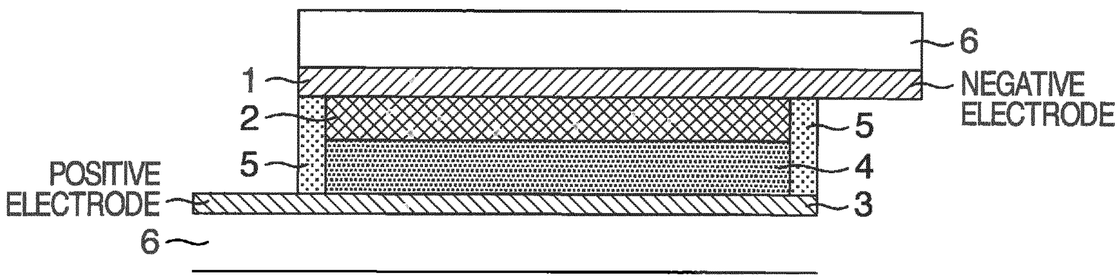


EP 2 928 009 A1

## Description

Technical Field

The present invention relates to a dye-sensitized solar cell which can achieve excellent photoelectric conversion efficiency and excellent durability.

Background Art

Solar cells which attract attention as a clean energy source have come to be used also for general housing in recent years, but have not yet sufficiently spread. One of the reasons includes that a module needs to be formed into a large size because the performance of a solar cell itself can hardly be said to be sufficiently excellent. Further, other reasons include that a solar cell itself is expensive because the productivity in the production of modules is low.

Although there are several types of solar cells, most solar cells put in practical use are silicon solar cells. However, a solar cell which has attracted attention recently and is investigated aiming at its practical utilization is a dye-sensitized solar cell. The prototype of the present dye-sensitized solar cell was developed by Dr. M. Graetzel (Switzerland) et al. in 1991 and is also called a Graetzel cell, The structure thereof is generally in the form in which a charge transfer layer (i.e. an electrolyte solution containing a redox material) is sandwiched between a semiconductor-containing layer made of dye-sensitized oxide semiconductor particulates provided on a transparent conductive substrate serving as one electrode and a substrate made of a counter electrode in which platinum or the like is arranged so as to face the semiconductor-containing layer. For example, a dye-sensitized solar cell has accomplished about the same performance as that of an amorphous-silicon solar cell by allowing a ruthenium complex dye to be adsorbed on a porous titanium oxide electrode (see Non Patent Literature 1). However, a large number of problems remain towards the practical utilization thereof. Particularly, improvement in durability in order to use it for a long period of time is one of the important problems that should be overcome. Further improvement in photoelectric conversion efficiency and durability of a dye-sensitized solar cell is desired also for achieving practical utilization of a dye-sensitized solar cell as a substitute of the silicon solar cell which is expensive in cost.

A sensitizing dye of a dye-sensitized solar cell is roughly classified into two families, ruthenium dyes and non-ruthenium dyes. In recent years, non-ruthenium dyes which have few restrictions on resources and are wide in the width of molecular design have been actively developed (see Non Patent Literature 2). Further, an electrode containing platinum is often used as a counter electrode of a dye-sensitized solar cell in order to achieve high conversion efficiency. Furthermore, an electrolyte solution containing an iodine redox couple having well-balanced performance is generally used as a charge transfer layer located between a semiconductor-containing layer and a counter electrode. However, although a dye-sensitized solar cell obtained by combining these components has good initial photoelectric conversion efficiency, this solar cell has the drawback of being poor in long-term stability due to high corrosiveness of an iodine redox system.

In order to solve the drawback as described above, Patent Literature 1 proposes a method of preparing a counter electrode by surface-treating a platinum substrate with a compound containing sulfur and a method of adding a sulfur-containing material to an electrolyte solution. Although the stability of the platinum counter electrode is surely improved by these methods, the surface treatment of the platinum substrate causes a large load on the process, and the treatment effect may not be sustained for a long period of time. Further, since a sulfur-containing material with a low oxidation number is generally oxidized by an iodine redox system, the possibility of significantly impairing the stability of an electrolyte solution is high when such a compound is added to the electrolyte solution. However, reference is not made at all to these drawbacks in the above patent. Furthermore, the durability evaluation results in examples are not at a level that would be considered to have a long-term stability of cells. Therefore, the method disclosed in the above patent must be said to be still an incomplete technique.

Further, similar examples of adding a sulfur material to an electrolyte solution include an electrolyte solution to which thiocyanate ions are added (see Non Patent Literature 3,

Patent Literature 2, and the like), an electrolyte solution to which an aminotriazole having an alkylthio group or a benzylthio group is added (see Patent Literature 3), and an electrolyte solution to which a sulfolane is added (see Patent Literature 4). However, none of these examples refer to the stability of an electrolyte solution or platinum, and the investigation of the configuration of a cell is not well developed. Particularly, when a generally widely known electrolyte solution to which thiocyanate ions are added is used, the possibility of impairing the long-term durability of cells is high. Thus, a prior art dye-sensitized solar cell using, as the components, all of a non-ruthenium dye, a platinum electrode, and an electrolyte solution containing an iodine redox system has been expected to be practically utilized, but still has a large number of disadvantages with respect to long-term durability.

Citation List

Patent Literatures

Patent Literature 1: WO 2012-014414
Patent Literature 2: JP 2010-192226 A
Patent Literature 3: IP 4264507 B
Patent Literature 4: WO 2009-069757

Non Patent Literatures

Non Patent Literature 1: Nature, vol. 353, pp. 737-740, 1991
Non Patent Literature 2: Chemical Reviews, vol. 110, No. 11, pp. 6616-6631, 2010
Non Patent Literature 3: The Journal of Physical Chemistry C, vol. 113, pp. 21779-21783, 2009

Summary of Invention

Technical Problem

An object of the present invention is to provide a dye-sensitized solar cell using a non-ruthenium dye which can achieve excellent photoelectric conversion efficiency and excellent durability.

Solution to Problem

As a result of intensive studies to achieve the above object, the present inventors have found that a dye-sensitized solar cell using a non-ruthenium dye as a sensitizing dye, platinum as a counter electrode, and an electrolyte solution containing iodine, iodide ions, and a compound having, in a molecule thereof, both a thioester bond and a positively charged nitrogen atom as a charge transfer layer is excellent in both photoelectric conversion efficiency and durability.

The aspects of the present invention are as follows.

[1] A dye-sensitized solar cell comprising: a first conductive support having a dye-sensitized semiconductor-containing layer; a second conductive support having a counter electrode provided in a position where the semiconductor-containing layer and the counter electrode are opposite to each other at a predetermined interval; a charge transfer layer sandwiched in a gap between the first and the second conductive supports; and a sealing agent provided in a peripheral part of the first and the second conductive supports in order to seal the charge transfer layer, wherein the dye is an organic non-ruthenium dye; the counter electrode contains platinum; and the charge transfer layer comprises an electrolyte solution containing iodine, iodide ions, and a compound having, in a molecule thereof, both a thioester bond and a positively charged nitrogen atom.

[2] The dye-sensitized solar cell according to the above item [1], wherein the compound having, in a molecule thereof, both a thioester bond and a positively charged nitrogen atom has a structure represented by formula (1):

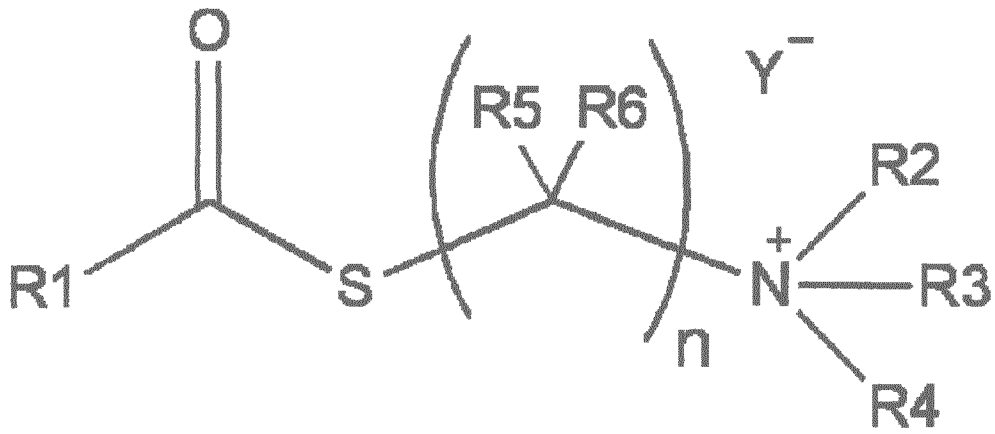

(1)

wherein R1, R2, R3, R4, R5, and R6 each independently represent an aliphatic hydrocarbon residue having 1 to 10 carbon atoms which may have one or more substituents selected from the group consisting of a halogen atom, an alkoxy group, an ester group, an acyl group, an amino group, an amide group, an alkyl group, an alkenyl group, an alkynyl group, an aryl group, a cyano group, an isocyano group, a nitro group, a nitroso group, a hydroxyl group, a phosphate group, a sulfinyl group, and a sulfonyl group, an aromatic hydrocarbon residue which may have one or more substituents selected from the group consisting of a halogen atom, an alkoxy group, an ester group, an acyl group, an amino group, an amide group, an alkyl group, an alkenyl group, an alkynyl group, an aryl group, a cyano group, an isocyano group, a nitro group, a nitroso group, a hydroxyl group, a phosphate group, a sulfinyl group, and a sulfonyl group, a heterocyclic residue which may have one or more substituents selected from the group consisting of a halogen atom, an alkoxy group, an ester group, an acyl group, an amino group, an amide group, an alkyl group, an alkenyl group, an alkynyl group, an aryl group, a cyano group, an isocyano group, a nitro group, a nitroso group, a hydroxyl group, a phosphate group, a sulfinyl group, and a sulfonyl group, or a hydrogen atom; any two selected from R1, R2, R3, R4, R5, and R6 may be combined to form a ring; n represents an integer of 1 to 6; and $Y^-$ represents a monovalent anion.

[3] The dye-sensitized solar cell according to the above item [2], wherein the compound represented by formula (1) is a compound having a thiocholine residue.

[4] The dye-sensitized solar cell according to the above item [2] or [3], wherein the compound represented by formula (1) is a compound having a halide ion.

[5] The dye-sensitized solar cell according to the above item [1], wherein the sealing agent is an epoxy resin sealing agent.

[6] The dye-sensitized solar cell according to the above item [1], wherein a semiconductor in the semiconductor-containing layer is titanium oxide in the form of particulates or a composite titanium oxide in the form of particulates.

[7] The dye-sensitized solar cell according to the above item [1], wherein the organic non-ruthenium dye has a structure represented by formula (2):

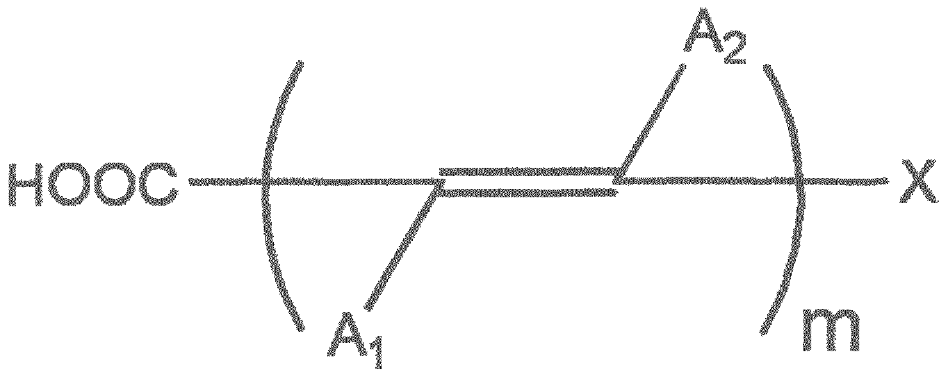

(2)

wherein $A_1$ and $A_2$ each independently represent a carboxyl group, a cyano group, an alkoxycarbonyl group, an acyl group, a nitro group, a cyclic hydrocarbon residue, a heterocyclic residue, an amino group, a hydroxyl group, a hydrogen atom, a halogen atom, or an alkyl group; X represents an aromatic hydrocarbon residue, a heterocyclic residue, or an amino group; m represents an integer of 1 to 6; when m is 2 or more and a plurality of $A_1$ and $A_2$ are present, each $A_1$ and each $A_2$ independently of each other represent said group, residue or atom which may be the

same or different; and any two of $A_1$ or each $A_1$ when a plurality of $A_1$ are present, $A_2$ or each $A_2$ when a plurality of $A_2$ are present, and X may be combined to form a ring.

[8] The dye-sensitized solar cell according to the above item [7], wherein $A_1$ in formula (2) is a cyano group or a carboxyl group.

[9] The dye-sensitized solar cell according to the above item [7] or [8], wherein X in formula (2) is a (poly)ethenyl group or a (poly)thiophenyl group each having a triphenylamine derivative.

Advantageous Effects of Invention

The dye-sensitized solar cell of the present invention is excellent in initial conversion efficiency and durability, particularly excellent in heat-resistant durability. This dye-sensitized solar cell can achieve high durability even in the case where an organic non-ruthenium dye is used as a sensitizing dye, and platinum is used as a counter electrode, the case being generally regarded as difficult in securing durability.

Brief Description of Drawings

[Fig. 1] Fig. 1 is a schematic sectional view illustrating a major portion of the structure of the dye-sensitized solar cell of the present invention.

Description of Embodiments

Hereinafter, the present invention will be described in detail.

The dye-sensitized solar cell of the present invention is a dye-sensitized solar cell comprising: a first conductive support having a dye-sensitized semiconductor-containing layer; a second conductive support having a counter electrode provided in a position where the semiconductor-containing layer and the counter electrode are opposite to each other at a predetermined interval; a charge transfer layer sandwiched in a gap between the first and the second conductive supports; and a sealing agent provided in a peripheral part of the first and the second conductive supports in order to seal the charge transfer layer, wherein the dye is an organic non-ruthenium dye; the counter electrode contains platinum; and the charge transfer layer comprises an electrolyte solution containing iodine, iodide ions, and a compound having, in a molecule thereof, both a thioester bond and a positively charged nitrogen atom.

The dye-sensitized solar cell of the present invention comprises a first conductive support having a dye-sensitized semiconductor-containing layer. The first conductive support is generally supported by a substrate formed of glass or the like.

Examples of the conductive support which is used include a conductive support in which a thin film of a conductive material typified by FTO (fluorine-doped tin oxide), ATO (antimony-doped tin oxide), and ITO (indium-doped tin oxide) is formed on the surface of a substrate such as glass, plastics, a polymer film, quartz, and silicon. The thickness of the substrate is generally 0.01 to 10 mm. Although the substrate can have various shapes from a film form to a sheet form, an optically transparent substrate is used in at least one of the first and the second conductive supports. The resistance of the conductive support is generally 1000 $\Omega/cm^2$ or less, preferably 100 $\Omega/cm^2$ or less.

Particulates of metal chalcogenides are preferred as an oxide semiconductor used for the preparation of the semiconductor-containing layer. Specific examples thereof include oxides of transition metals such as Ti, Zn, Sn, Nb, W, In, Zr, Y, La, and Ta, oxides of aluminum, oxides of Si, and perovskite-type oxides such as $StTiO_3$, $CaTiO_3$, and $BaTiO_3$. Among these oxides, $TiO_2$, ZnO, and $SnO_2$ are particularly preferred. Further, these oxides may be used in combination, and preferred examples include a $SnO_2$-ZnO mixed system. In the case of a mixed system, components may be mixed in the state of fine particles or in the state of a slurry or a paste to be described below, or each component may be stacked in layers and used. The primary particle size of the oxide semiconductor used here is generally 1 to 200 nm, preferably 1 to 50 nm. Further, for the purpose of improving the open circuit voltage and conversion efficiency of a dye-sensitized solar cell, it is also possible to use, as the oxide semiconductor, a composite oxide semiconductor prepared by mixing titanium with a non-titanium metal such as magnesium, calcium, zirconium, and strontium or the like, which is described, for example, in WO 2006/080384.

The sensitizing dye which can be used in the dye-sensitized solar cell of the present invention is not particularly limited as long as it is an organic non-ruthenium dye which has an action of sensitizing the optical absorption in cooperation with semiconductor particulates constituting the semiconductor-containing layer. The organic non-ruthenium dyes may be used as a single dye or as a mixture in which several types of dyes are mixed in an arbitrary ratio. When the organic non-ruthenium dyes are used as a mixture in which several types of dyes are mixed, a solar cell having high conversion efficiency is obtained because a wide absorption wavelength can be used by mixing dyes each having a different absorption wavelength region.

Specific examples of the organic non-ruthenium dyes include methine dyes such as cyanine dyes, merocyanine

dyes, oxonol dyes, triphenylmethane dyes, acrylic acid dyes described in WO 2002/011213, and pyrazolone methine dyes described in WO 2006/126538, xanthene dyes, azo dyes, anthraquinone dyes, perylene dyes, indigo dyes, acridine dyes, quinone dyes, coumarin dyes, phenyl xanthene dyes, phthalocyanine dyes in which central metal is not ruthenium, and porphyrin dyes in which central metal is not ruthenium. Among these dyes, preferred are dyes described in JP3731752 B, JP 2002-334729 A, JP 2002-512729 A, JP 2003-007358 A, JP 2003-017146 A, JP 2003-059547 A, JP 2003-086257 A, JP 2003-115333 A, JP 2003-132965 A, JP 2003-142172 A, JP 2003-151649 A, JP 2003-157915 A, JP 2003-282165 A, JP 2004-014175 A, JP 2004-022222 A, JP 2004-022387 A, JP 2004-227825 A, JP 2005-005026 A, JP 2005-019130 A, JP 2005-135656 A, JP 2006-079898 A, JP 2006-134649 A, JP 2007-149570 A, JP 2008-021496 A, JP 2010-146864 A, WO 2002/001667, WO 2002/011213, WO 2002/071530, WO 2004/082061, WO 2006/082061, WO 2006/126538, WO 2007/100033, WO 2009/020098, WO 2010/021378, and the like. Especially, methine dyes such as merocyanine dyes and acrylic acid dyes are more preferred

Among these sensitizing dyes, a dye represented by the following formula (2) is particularly preferably used in the dye-sensitized solar cell of the present invention.

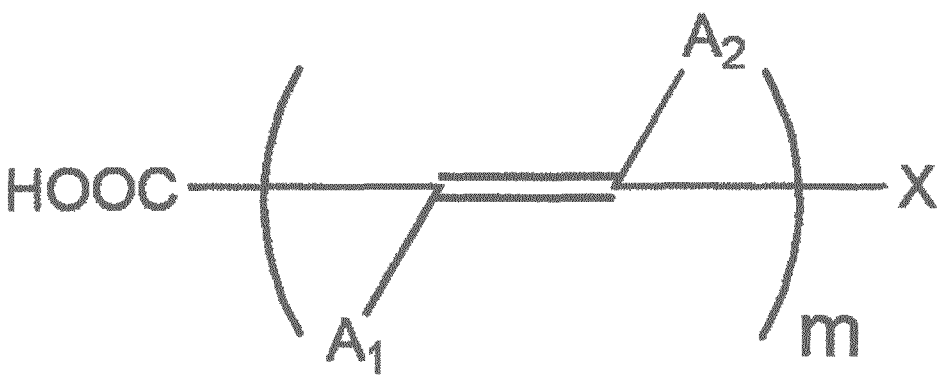

(2)

The term "a dye represented by formula (2)" referred to herein means that both a free acid represented by the above formula (2) and a salt thereof are included, unless otherwise specified. Examples of the salt of a dye represented by formula (2) can include a compound in which the carboxylic acid part in formula (2) forms a metal salt with an alkali metal or an alkaline earth metal such as lithium, sodium, potassium, magnesium, or calcium, or forms a quaternary ammonium salt with tetramethylammonium, tetrabutylammonium, pyridinium, imidazolium, or the like.

In formula (2), $A_1$ and $A_2$ each independently represent a carboxyl group, a cyano group, an alkoxycarbonyl group, an acyl group, a nitro group, a cyclic hydrocarbon residue, a heterocyclic residue, an amino group, a hydroxyl group, a hydrogen atom, a halogen atom, or an alkyl group. Further, when a plurality of $A_1$ and $A_2$ are present, each $A_1$ and each $A_2$ independently of each other represent the group, residue or atom which may be the same or different.

Examples of the alkyl group of the alkoxycarbonyl group represented by $A_1$ and $A_2$ in formula (2) include an optionally substituted saturated or unsaturated linear, branched or cyclic alkyl group. The linear or branched alkyl group is preferably an alkyl group having 1 to 36 carbon atoms, more preferably a saturated linear alkyl group having 1 to 20 carbon atoms. Examples of the cyclic alkyl group include a cycloalkyl group having 3 to 8 carbon atoms.

Examples of the acyl group represented by $A_1$ and $A_2$ in formula (2) include an alkylcarbonyl group having I to 10 carbon atoms and an arylcarbonyl group. Preferred examples include an alkylcarbonyl group having 1 to 4 carbon atoms. Specific examples include an acetyl group and a propionyl group

The cyclic hydrocarbon residue represented by $A_1$ and $A_2$ in formula (2) means a group obtained by removing one hydrogen atom from a cyclic hydrocarbon. Examples of the cyclic hydrocarbon include a benzene ring, a naphthalene ring, an anthracene ring, a phenanthrene ring, a pyrene ring, an indene ring, an azulene ring, a fluorene ring, a cyclohexane ring, a cyclopentane ring, a cyclohexene ring, a cyclopentene ring, a cyclohexadiene ring, and a cyclopentadiene ring.

The cyclic hydrocarbon residue represented by $A_1$ and $A_2$ may have a substituent, and examples of the substituent include an alkyl group, an aryl group, a cyano group, an isocyano group, a thiocyanate group, an isothiocyanato group, a nitro group, a nitrosyl group, an acyl group, a halogen atom, a hydroxyl group, a phosphoric acid group, a phosphate group, a substituted or unsubstituted mercapto group, a substituted or unsubstituted amino group, a substituted or unsubstituted amide group, an alkoxy group, an alkoxyalkyl group, a carboxyl group, an alkoxycarbonyl group, and a sulfo group. Examples of the alkyl group as described here include the same alkyl group as that of the above-described alkoxycarbonyl group. Examples of the acyl group include the same acyl group as described above, and examples of the aryl group include groups obtained by removing a hydrogen atom from the aromatic rings listed in the above-described paragraph of the cyclic hydrocarbon residue. The aryl group may further have a substituent, and examples of the substituent include those which the above-described cyclic hydrocarbon residue may have. Examples of the halogen atom include a chlorine atom, a bromine atom, and an iodine atom. Examples of the phosphate group include a C1-4

alkyl phosphate group. Examples of the substituted or unsubstituted mercapto group include a mercapto group and an alkyl mercapto group. Examples of the substituted or unsubstituted amino group include an amino group, mono- or dialkylamino group, and mono- or diaromatic amino group. Specific examples include mono- or dimethylamino group, mono- or dimethylamino group, mono- or dipropylamino group, mono- or diphenylamino group, and mono-or dibenzylamino group. Examples of the substituted or unsubstituted amide group include an amide group, an alkylamide group, and an aromatic amide group. Examples of the alkoxy group include an alkoxy group having 1 to 10 carbon atoms. Examples of the alkoxyalkyl group include a C1-10 alkoxy C1-4 alkyl group. Examples of the alkoxycarbonyl group include an alkoxycarbonyl group having 1 to 10 carbon atoms. Further, the acidic group such as a carboxyl group, a sulfo group, and a phosphoric acid group may form a salt of a metal such as lithium, sodium, potassium, magnesium, and calcium and a salt of quaternary ammonium such as tetramethylammonium, tetrabutylammonium, pyridinium, and imidazolium.

The heterocyclic residue represented by $A_1$ and $A_2$ in formula (2) means a group obtained by removing one hydrogen atom from a heterocyclic compound. Examples of the heterocyclic compound include a pyridine ring, a pyrazine ring, a pyrimidine ring, a pyrazole ring, a pyrazolidine ring, a piperidine ring, a thiazolidine ring, an oxazolidine ring, a pyran ring, a chromene ring, a pyrrole ring, a benzimidazole ring, an imidazoline ring, an imidazolidine ring, an imidazole ring, a pyrazole ring, a triazole ring, a triazine ring, a diazole ring, a morpholine ring, an indoline ring, a thiophene ring, a bithiophene ring, a terthiophene ring, a furan ring, an oxazole ring, a thiazine ring, a thiazole ring, an indole ring, a benzothiazole ring, a naphthothiazole ring, a benzoxazole ring, a naphthoxazole ring, a indolenine ring, a benzoindolenine ring, a pyrazine ring, a quinoline ring, a quinazoline ring, and a carbazole ring. These rings each may be annulated or hydrogenated. The heterocyclic residue may have a substituent, and examples of the substituent include those which the above-described cyclic hydrocarbon residue may have.

Preferred examples of the heterocyclic residue represented by $A_1$ and $A_2$ include groups obtained by removing one hydrogen atom from a heterocyclic compound such as a pyridine ring, a pyrazine ring, a piperidine ring, a morpholine ring, an indoline ring, a thiophene ring, a furan ring, an oxazole ring, a thiazole ring, an indole ring, a benzothiazole ring, a benzoxazole ring, a pyrazine ring, and a quinoline ring.

The amino group represented by $A_1$ and $A_2$ in formula (2) may have a substituent Examples of the amino group having a substituent include a mono- or dialkylamino group, a mono- or diaromatic amino group, and a monoalkyl monoaromatic amino group, and examples of the alkyl group in the alkylamino group include the same alkyl group as that of the above-described alkoxycarbonyl group. Further, examples of the aromatic residue of the aromatic amino group include the same cyclic hydrocarbon residue as described above. Specific examples of the amino group having a substituent include a mono- or dimethylamino group, a mono- or diethylamino group, a mono- or dipropylamino group, a mono- or diphenylamino group, and a mono- or dibenzylamino group.

Examples of the halogen atom represented by $A_1$ and $A_2$ in formula (2) include the same halogen atom as described above.

Examples of the alkyl group represented by $A_1$ and $A_2$ in formula (2) include the same alkyl group as that of the above-described alkoxycarbonyl group. This alkyl group may have a substituent. Examples of the substituent which the alkyl group may have include an aryl group, a halogen atom, and an alkoxy group. Examples of the aryl group and the halogen atom as described here include the same aryl group and halogen atom as described above, and examples of the alkyl group of the alkoxy group include the same alkyl group as that of the alkoxycarbonyl group.

Further, both $A_1$ and $A_2$ may be combined to form a ring. Particularly, in the case to be described below where m is 2 or more and a plurality of a and a plurality of $A_2$ are present, any two thereof may be combined to form a ring. When a ring is formed, it is not particularly limited whether which $A_1$ is combined with which $A_2$, but generally, $A_1$ and $A_2$ located adjacent to each other, $A_1$ and $A_1$ located adjacent to each other, or $A_2$ and $A_2$ located adjacent to each other form a ring. This ring may have a substituent. Examples of the substituent when the ring has a substituent include those which the above-described cyclic hydrocarbon residue may have. Examples of the ring to be formed by the combination of $A_1$ and $A_2$, or any two of a plurality of $A_1$ and a plurality of $A_2$ include an unsaturated hydrocarbon ring or a heterocyclic ring. Examples of the unsaturated hydrocarbon ring include a benzene ring, a naphthalene ring, an anthracene ring, a phenanthrene ring, a pyrene ring, an indene ring, an azulene ring, a fluorene ring, a cyclobutene ring, a cyclopentene ring, a cyclohexene ring, a cyclohexadiene ring, and a cyclopentadiene ring. Examples of the heterocyclic ring include a pyridine ring, a pyrazine ring, an indoline ring, a thiophene ring, a furan ring, a pyran ring, an oxazole ring, a thiazole ring, an indole ring, a benzothiazole ring, a benzoxazole ring, a pyrazine ring, a quinoline ring, a carbazole ring, and a benzopyran ring. Preferred examples of these rings include a cyclobutene ring, a cyclopentene ring, a cyclohexene ring, and a pyran ring. Further, when $A_1$ or $A_2$ has a carbonyl group or a thionyl group, a cyclic ketone or a cyclic thioketone may be formed.

Preferred examples of $A_1$ and $A_2$ independently include a carboxyl group, a cyano group, an alkoxycarbonyl group, an acyl group, a hydroxyl group, a hydrogen atom, a halogen atom, and an alkyl group. More preferred examples include a carboxyl group, a cyano group, a hydrogen atom, a halogen atom, and an alkyl group. Among the halogen atom, a chlorine atom, a bromine atom, and an iodine atom are preferred. Further, $A_1$ attacked to the same carbon atom

to which the carboxyl group clearly shown in formula (2) is attached ($A_1$ nearest to the clearly-shown carboxyl group) is particularly preferably a carboxyl group or a cyano group.

In formula (2), m represents an integer of 1 to 6.

X represents an aromatic hydrocarbon residue, a heterocyclic residue, or an amino group. The aromatic hydrocarbon residue means a group obtained by removing one hydrogen atom from an aromatic hydrocarbon. Examples of the aromatic hydrocarbon residue include a group obtained by removing one hydrogen atom from an aromatic hydrocarbon such as a benzene ring, a naphthalene ring, an anthracene ring, a phenanthrene ring, a pyrene ring, an indene ring, an azulene ring, and a fluorene ring. These aromatic hydrocarbon residues each generally have an aromatic ring (such as an aromatic ring and a condensed ring containing an aromatic ring) having 6 to 16 carbon atoms. These aromatic hydrocarbon residues each may have a substituent.

Examples of the heterocyclic residue represented by X in formula (2) include a group obtained by removing one hydrogen atom from a heterocyclic compound. Examples of the heterocyclic compound include a pyridine ring, a pyrazine ring, a pyrimidine ring, a pyrazole ring, a pyrazolidine ring, a thiazolidine ring, an oxazolidine ring, a pyran ring, a chromene ring, a pyrrole ring, a benzimidazole ring, an imidazoline ring, an imidazolidine ring, an imidazole ring, a pyrazole ring, a triazole ring, a triazine ring, a diazole ring, a morpholine ring, an indoline ring, a thiophene ring, a bithiophene ring, a terthiophene ring, a furan ring, an oxazole ring, a thiazine ring, a thiazole ring, an indole ring, a benzothiazole ring, a naphthothiazole ring, a benzoxazole ring, a naphthoxazole ring, an indolenine ring, a benzoindolenine ring, a pyrazine ring, a quinoline ring, a quinazoline ring, and a carbazole ring. These rings each may be annulated or hydrogenated, and each may have a substituent. Further, when X is a heterocyclic residue, the heterocyclic ring may be quaternized, and may have a counter ion at this time. The counter ion may be a common anion, but is not particularly limited thereto. Specific examples thereof include a fluoride ion, a chloride ion, a bromide ion, an iodide ion, a perchlorate ion, a hydroxide ion, a methylsulfate ion, a toluenesulfonate anion, a tetrafluoroborate anion, a hexafluorophosphonate anion, a thiocyanate anion, a tetracyanoborate anion, a dicyanoimide anion, a trifluoromethanesulfonate anion, a bis(trifluoromethanesulfonyl)imide anion, a bis(pentafluoroethanesulfonyl)imide anion, and an (N-trifluoromethanesulfonyl-N-pentafluoroethanesulfonyl)imide anion. A bromide ion, an iodide ion, a perchlorate ion, a tetrafluoroborate anion, a hexafluorophosphonate anion, a toluenesulfonate anion, a trifluoromethanesulfonate anion, and a bis(trifluoromethanesulfonyl)imide anion are preferred. Alternatively, the quaternized heterocyclic ring may be neutralized with an intramolecular or intermolecular acidic group such as a carboxyl group instead of a counter ion.

The amino group represented by X in formula (2) may have a substituent. Specific examples of the optionally substituted amino group include an amino group, a diphenylamino group, a monophenylamino group, a dialkylamino group, a monoalkylamino group, an alkylphenylamino group, an alkoxyamino group, and an acylamino group (such as a benzoylamino group and an acetylamino group). Examples of the alkyl group, the alkoxy group, and the acyl group of these amino groups include the same ones as described above.

X may be combined with $A_1$ or $A_2$ to form a ring, and the ring formed may have a substituent. Examples of the ring which is formed by the combination of X with $A_1$ or $A_2$, include a benzene ring, a naphthalene ring, an indene ring, a pyridine ring, a pyrazine ring, a pyrimidine ring, a quinoline ring, a thiophene ring, an indolenine ring, a benzoindolenine ring, a pyrazole ring, a pyrazolidine ring, a thiazole ring, a thiazolidine ring, a benzothiazole ring, an oxazole ring, an oxazolidine ring, a benzoxazole ring, a pyran ring, a chromene ring, a pyrrole ring, an imidazole ring, a benzimidazole ring, an imidazoline ring, an imidazolidine ring, an indole ring, a furan ring, a carbazole ring, a pyran ring, a benzopyran ring, a phthalocyanine ring, a porphyrin ring, and ferrocene. These rings may be hydrogenated.

Examples of the substituent when the aromatic hydrocarbon residue or the heterocyclic residue in X has a substituent and examples of the substituent when a ring formed from two of X and $A_1$ or $A_2$ described above has a substituent thereon include the same substituent as that of the cyclic hydrocarbon as described in the previous paragraph of $A_1$ or $A_2$, a carbonyl group, and a thiocarbonyl group. Further, when X and $A_1$ or $A_2$ forming a ring has a carbonyl group or a thiocarbonyl group, the ring formed from two of X and $A_1$ or $A_2$ may be a ring substituted with O= or S= as a substituent, that is, a cyclic ketone or a cyclic thioketone. Preferred examples of the substituent in the above-described aromatic hydrocarbon residue or heterocyclic residue in X and the substituent on a ring formed from two of X and $A_1$ or $A_2$ include an optionally substituted amino group, an optionally substituted alkyl group, an optionally substituted alkoxy group, an optionally substituted acetyl group, a hydroxyl group, a halogen atom, O=, and S=. More preferred examples thereof include an optionally substituted amino group, an optionally substituted alkyl group, an optionally substituted alkoxy group, O=, and S=. Here, examples of the optionally substituted amino group include a mono- or dialkyl-substituted amino group, a monoalkyl monoaryl-substituted amino group, a diaryl-substituted amino group, a mono- or divinyl-substituted amino group, a mono- or diallyl-substituted amino group, a mono- or dibutadienyl-substituted amino group, and a mono- or distyryl-substituted amino group. Among them, a dialkyl-substituted amino group and a diaryl-substituted amino group are preferred. Examples of the optionally substituted alkyl group include an aryl-substituted alkyl group, a halogen atom-substituted alkyl group, and an alkoxy-substituted alkyl group. Examples of the optionally substituted alkoxy group include an alkoxy-substituted alkoxy group, a halogen-substituted alkoxy group, and an aryl-substituted alkoxy group.

Particularly preferred examples of X include an ethenyl group derivative, a butadienyl group derivative, a hexatrienyl group derivative, a thiophenyl group derivative, a bithiophenyl group derivative, and a terthiophenyl group derivative, each having a triphenylamine derivative at a terminal. These derivatives each may have a substituent. This substituent may be the same substituent as that listed above in the case where the aromatic hydrocarbon residue or heterocyclic residue in X has a substituent. X is particularly preferably a (poly)ethenyl group or a (poly)thiophenyl group each having a triphenylamine derivative.

The dye represented by formula (2) can take a structural isomer such as cis-form and trans-form. Both structural isomers can be satisfactorily used as a dye for photosensitization without any particular limitation.

Specific examples of such a sensitizing dye include dyes described in WO 2002/011213, JP 2003-017146 A, JP 2003-282165 A, WO 2004/082061, JP 2006-134649 A, JP 2006-079898 A, WO 2007/100033, and JP 2007-149570 A.

The dye-sensitized solar cell of the present invention comprises a second conductive support having a counter electrode.

The surface of the same conductive support as that used in the first conductive support is vapor-deposited with platinum, as a counter electrode, which catalytically acts on a reduction reaction of a redox electrolyte, or coated with metal particulates containing platinum or a precursor of metal particulates containing platinum followed by firing, to obtain a conductive support which is used as the second conductive support.

The dye-sensitized solar cell of the present invention comprises, as a charge transfer layer, an electrolyte solution containing iodine, iodide ions, and a compound having, in a molecule thereof, both a thioester bond and a positively charged nitrogen atom. A compound having any structure may be used in the dye-sensitized solar cell of the present invention as long as the compound having, in a molecule thereof, both a thioester bond and a positively charged nitrogen atom is a compound having, in a molecule thereof, at least one thioester bond and at least one positively charged nitrogen atom. The thioester bond has a structure in which carboxylic acid and thiol have undergone dehydration condensation, and can be represented by the chemical formula of R-CO-S-R'. Further, the positively charged nitrogen atom is a nitrogen atom having four covalent bonds, and specific examples thereof include a quaternary ammonium cation, an iminium cation, and a cationic nitrogen-containing heterocyclic ring such as pyridinium, imidazolium, pyrrolidinium, pyrrolium, pyrazolium, and oxazolium. Further, the positively charged nitrogen atom may have any counter ion. Examples of the counter ion include halonium ions, oxo anions, thiocyanate ions, borate ions, imide ions, sulfonate ions, and metal complex ions of metals such as aluminum, chromium, silver, zinc, and iron.

Among the compounds each having, in a molecule thereof, both a thioester bond and a positively charged nitrogen atom, a compound represented by the following formula (1) is more preferably used in the dye-sensitized solar cell of the present invention.

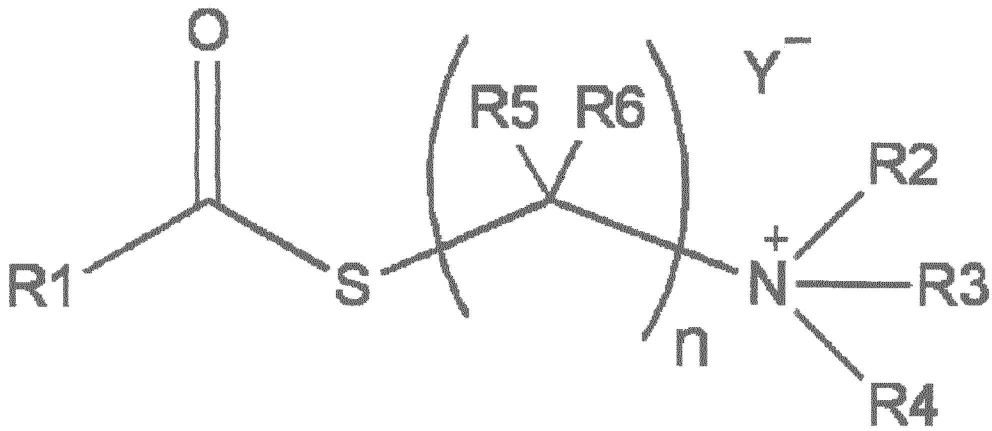

(1)

In formula (1), R1, R2, R3, R4, R5, and R6 each independently represent an optionally substituted aliphatic hydrocarbon residue having 10 or less carbon atoms, an optionally substituted aromatic hydrocarbon residue, an optionally substituted heterocyclic residue, or a hydrogen atom. Further, when n is 2 or more, and a plurality of R5 and R6 are present, each R5 and each R6 independently of each other represent the above residue or atom which may be the same or different.

The aliphatic hydrocarbon residue having 1 to 10 carbon atoms represented by R1 to R6 means a residue obtained by removing one hydrogen atom from an aliphatic hydrocarbon having 1 to 10 carbon atoms. The aliphatic hydrocarbon residue may be linear, branched, or cyclic, and may be a saturated aliphatic hydrocarbon or an unsaturated aliphatic hydrocarbon. Further, the aliphatic hydrocarbon residue having 1 to 10 carbon atoms may have a substituent selected from the group consisting of, for example, a halogen atom, an alkoxy group, an ester group, an acyl group, an amino group, an amide group, an alkyl group, an alkenyl group, an alkynyl group, an aryl group, a cyano group, an

isocyano group, a nitro group, a nitroso group, a hydroxyl group, a phosphate group, a sulfonyl group, and a sulfonyl group. The position and the number of substitution of the substituent are not particularly limited. These aliphatic hydrocarbon residues may have a plurality of the same substituents or a plurality of different substituents.

The aromatic hydrocarbon residue represented by R1 to R6 means a residue obtained by removing one hydrogen atom from an aromatic hydrocarbon. Examples of the aromatic hydrocarbon include a benzene ring, a naphthalene ring, an anthracene ring, a phenanthrene ring, a pyrene ring, an indene ring, an azulene ring, and a fluorene ring. These rings each may be annulated. Further, the aromatic hydrocarbon residue may have a substituent selected from the group consisting of, for example, a halogen atom, an alkoxy group, an ester group, an acyl group, an amino group, an amide group, an alkyl group, an alkenyl group, an alkynyl group, an aryl group, a cyano group, an isocyano group, a nitro group, a nitroso group, a hydroxyl group, a phosphate group, a sulfinyl group, and a sulfinyl group. The position and the number of substitution of the substituent are not particularly limited. These aromatic hydrocarbon residues may have a plurality of the same substituents or a plurality of different substituents.

The heterocyclic residue represented by R1 to R6 means a residue obtained by removing one hydrogen atom from a heterocyclic compound. Examples of the heterocyclic compound include a pyrrolidine ring, an oxolane ring, a thiolane ring, a pyrrole ring, a furan ring, a thiophene ring, a piperidine ring, an oxane ring, a thiane ring, a pyridine ring, an imidazole ring, a pyrazole ring, an oxazole ring, a thiazole ring, an imidazoline ring, a pyrazine ring, a morpholine ring, a thiazine ring, an indole ring, an isoindole ring, a benzimidazole ring, a purine ring, a quinoline ring, an isoquinoline ring, a quinoxaline ring, a cinnoline ring, a pteridine ring, a chromene ring, an isochromene ring, an acridine ring, a xanthene ring, and a carbazole ring. These rings each may be annulated or hydrogenated. Further, the heterocyclic residue may have a substituent selected from the group consisting of, for example, a halogen atom, an alkoxy group, an ester group, an acyl group, an amino group, an amide group, an alkyl group, an alkenyl group, an alkynyl group, an aryl group, a cyano group, an isocyano group, a nitro group, a nitroso group, a hydroxyl group, a phosphate group, a sulfonyl group, and a sulfonyl group. The position and the number of substitution of the substituents are not particularly limited. These heterocyclic residues may have a plurality of the same substituents or a plurality of different substituents.

Further, any two selected from R1, R2, R3, R4, R5, and R6 may be combined to form a ring. When n is 2 or more, and a plurality of R5 and R6 are present, the plurality of R5 may form a ring, and the plurality of R6 may form a ring. The ring which may be formed may be any of a saturated hydrocarbon ring, an unsaturated hydrocarbon ring, a saturated heterocyclic ring, and an unsaturated heterocyclic ring. Further, the ring which may be formed may have any number of substituents at any position. Examples of the ring which may be formed include a cyclohexane ring, a cyclopentane ring, a cyclohexene ring, a cyclopentene ring, a cyclohexadiene ring, a cyclopentadiene ring, a lactone ring, a lactam ring, a cyclic ketone, a benzene ring, a naphthalene ring, an anthracene ring, a phenanthrene ring, a pyrene ring, an indene ring, an azulene ring, a fluorene ring, a pyrrolidine ring, an oxolane ring, a thiolane ring, a pyrrole ring, a furan ring, a thiophene ring, a piperidine ring, an oxane ring, a thiane ring, a pyridine ring, an imidazole ring, a pyrazole ring, an oxazole ring, a triazole ring, an imidazoline ring, a pyrazine ring, a morpholine ring, a thiazine ring, an indole ring, an isoindole ring, a benzimidazole ring, a purine ring, a quinoline ring, an isoquinoline ring, a quinoxaline ring, a cinnoline ring, a pteridine ring, a chromene ring, an isochromene ring, an acridine ring, a xanthene ring, and a carbazole ring. These rings each may be annulated or hydrogenated. Examples of the substituent which may be possessed include a halogen atom, an alkoxy group, an ester group, an acyl group, an amino group, an amide group, an alkyl group, an alkenyl group, an alkynyl group, an aryl group, a cyano group, an isocyano group, a nitro group, a nitroso group, a hydroxyl group, a phosphate group, a sulfinyl group, and a sulfonyl group.

Preferred examples of R1, R2, R3, R4, R5, and R6 include an aliphatic hydrocarbon residue having 1 to 6 carbon atoms, a phenyl group, a naphthalenyl group, a benzyl group, a pyridyl group, a pyrrole group, a thiophenyl group, a furanyl group, an oxolanyl group, an oxyanyl group, a substituent in which a hydrogen atom in these substituents is replaced with an alkyl group, an aryl group, an alkenyl group, an alkynyl group, an alkoxy group, an ester group, an acyl group, an amino group, an amide group, a halogen atom, a cyano group, or the like, and a hydrogen atom.

In formula (1), $Y^-$ represents a monovalent anion serving as a counter ion of a nitrogen cation. $Y^-$ is not particularly limited as long as it is a monovalent anion which can be stably present in an iodine electrolyte solution. An anion having low basicity is preferred. Preferred examples of the anion include a fluoride ion, a chloride ion, a bromide ion, an iodide ion, a perchlorate ion, a hydroxide ion, a methyl sulfate ion, a toluenesulfonate anion, a tetrafluoroborate anion, a hexafluorophosphonate anion, a tetracyanoborate anion, a dicyanoimide anion, a trifluoromethanesulfonate anion, a bis(trifluoromethanesulfonyl)imide anion, a bis(pentafluoroethanesulfonyl)imide anion, and an (N-trifluoromethanesulfonyl-N-pentafluoroethanesulfonyl)imide anion. Among them, a chloride ion, a bromide ion, an iodide ion, a perchlorate ion, a tetrafluoroborate anion, a hexafluorophosphonate anion, a trifluoromethanesulfonate anion, a bis(trifluoromethanesulfonyl)imide anion, a bis(pentafluoroethanesulfonyl)imide anion, and an (N-trifluoromethanesulfonyl-N-pentafluoroethanesulfonyl)imide anion are more preferred.

In formula (1), n represents an integer of 1 to 6, preferably an integer of 1 to 4, more preferably an integer of 1 to 3.

Among the compounds represented by formula (1), a compound having a thiocholine residue in which n = 2 and both R5 and R6 are each a hydrogen atom is particularly preferably used in the dye-sensitized solar cell of the

present invention. Further, in the compounds of formula (1) having a thiocholine residue, R1, R2, R3, and R4 are preferably an aliphatic hydrocarbon residue having 1 to 6 carbon atoms, a phenyl group, a naphthalenyl group, a benzyl group, a pyridyl group, a pyrrole group, a thiophenyl group, a furanyl group, an oxolanyl group, an oxyanyl group, a substituent in which a hydrogen atom in these substituents is replaced with an alkyl group, an alkenyl group, an alkynyl group, an aryl group, an alkoxy group, an ester group, an acyl group, an amino group, an amide group, a halogen atom, a cyano group, or the like, and a hydrogen atom, as described above. R1, R2, R3, and R4 are more preferably a methyl group, an ethyl group, a propyl group, a butyl group, a phenyl group, a benzyl group, a thiophenyl group, a furanyl group, and a substituent in which a hydrogen atom in these substituents is replaced with any of an alkoxy group, an ester group, an acyl group, an amide group, and a halogen atom. Further, in the compounds of formula (1) having a thiocholine residue, $Y^-$ is preferably a fluoride ion, a chloride ion, a bromide ion, an iodide ions, a perchlorate ion, a hydroxide ion, a methylsulfate ion, a toluenesulfonate anion, a tetrafluoroborate anion, a hexafluorophosphonate anion, a tetracyanoborate anion, a dicyanoimide anion, a trifluormethanesulfonate anion, a bis(trifluoromethanesulfonyl)imide anion, a bis(pentafluoroethanesulfonyl)imide anion, and an (N-trifluoromethanesulfonyl-N-pentafluoroethanesulfonyl)imide anion, as described above. $Y^-$ is more preferably a chloride ion, a bromide ion, an iodide ion, a perchlorate ion, a tetrafluoroborate anion, a hexafluorophosphonate anion, a trifluoromethanesulfonate anion, a bis(trifluoromethanesulfonyl)imide anion, a bis(pentafluoroethanesulfonyl)imide anion, and an (N-trifluoromethanesulfonyl-N-pentafluoroethanesulfonyl)imide anion. Among them, the compound represented by formula (1) is most preferably a chloride, a bromide, and an iodide of acetylthiocholine, propionylthiocholine, butyrylthiocholine, and benzoylthiocholine.

The compound having, in a molecule thereof, both a thioester bond and a positively charged nitrogen atom in the dye-sensitized solar cell of the present invention may be used singly or in combination. These compounds may be a commercially available compound or an originally synthesized compound. A compound having high purity is more preferred, and a compound having high solubility in an electrolyte solvent is more suitable. The concentration of these compounds in an electrolyte solution is generally 0.01 to 2 M, preferably 0.02 to 1 M, more preferably 0.03 to 0.5 M, particularly preferably 0.05 to 0.3 M.

The electrolyte solution of the dye-sensitized solar cell of the present invention generally contains a compound having an iodide ion as a counter ion which can release the iodide ion in the electrolyte solution. The compound having an iodide ion as a counter ion is not particularly limited as long as it is a compound which can provide iodide ions in an electrolyte solution. A compound having a high degree of dissociation of iodide ions is preferred. Preferred examples of the compound having an iodide ion as a counter ion include halogenated metallic salts such as lithium iodide, sodium iodide, potassium iodide, and cesium iodide; ammonium iodides such as trimethylammonium iodide, tetrapropylammonium iodide, and tetrabutylammonium iodide; imidazolium iodides such as imidazolium iodide, 1,3-dimethylimidazolium iodide, 1-ethyl-3-methylimidazolium iodide, 1-methyl-3-propylimidazolium iodide, 1-butyl-3-methylimidazolium iodide, 1-hexyl-3-methylimidazolium iodide, 1,2-dimethyl-3-propylimidazolium iodide, 1,2-dimethyl-3-butylimidazolium iodide, and 1,2-dimethyl-3-hexylimidazolium iodide; pyrrolidinium iodides such as N,N-dimethylpyrrolidinium iodide, N-methyl-N-propylpyrrolidinium iodide, and N,N-dibutylpyrrolidinium iodide; pyridinium iodides such as N-methylpyridinium iodide, N-propylpyridinium iodide, and N-butylpyridinium iodide; pyrrolium iodides such as 1-ethyl-1-methylpyrrolium iodide; pyrazolium iodides such as 1-propyl-2-methylpyrazolium iodide; and phosphonium iodides such as tetrabutylphosphonium iodide. Among the compounds each having an iodide ion as a counter ion, lithium iodide, sodium iodide, potassium iodide, trimethylammonium iodide, tetrabutylammonium iodide, 1,3-dimethylimidazolium iodide, 1-ethyl-3-methylimidazolium iodide, 1-methyl-3-propylimidazolium iodide, 1-butyl-3-methylimidazolium iodide, and 1,2-dimethyl-3-propylimidazolium iodide are more preferred. The compounds each having an iodide ion as a counter ion may be used singly or in combination in the electrolyte solution of the dye-sensitized solar cell of the present invention. The concentration of these compounds in the electrolyte solution is generally 0.01 to 10 M, preferably 0.02 to 5 M, more preferably 0.03 to 3 M, particularly preferably 0.05 to 2 M.

An electrochemically inert solvent may be used in combination in the electrolyte solution of the dye-sensitized solar cell of the present invention. The solvent which can be used in combination may be any of an organic solvent and an ionic liquid or may be a mixture thereof. Preferred examples of the organic solvent which can be used in combination include acetonitrile, butyronitrile, valeronitrile, hexanenitrile, propylene carbonate, ethylene carbonate, 3-methoxypropionitrile, methoxyacetonitrile, ethylene glycol, propylene glycol, diethylene glycol, triethylene glycol, diethylene glycol dimethyl ether, triethylene glycol dimethyl ether, tetraethylene glycol dimethyl ether, 1,2-dimethoxyethane, γ-butyrolactone, diethyl ether, diethyl carbonate, dimethyl carbonate, dimethylformamide, dimethyl sulfoxide, 1,3-dioxolane, methyl formate, 2-methyltetrahydrofuran, 3-methyl-oxazolidin-2-one, sulfolane, tetrahydrofuran, and methylisopropylsulfone. Among these organic solvents, acetonitrile, valeronitrile, hexanenitrile, propylene carbonate, ethylene carbonate, 3-methoxypropionitrile, methoxyacetonitrile, diethylene glycol dimethyl ether, triethylene glycol dimethyl ether, 1,2-dimethoxyethane, γ-butyrolactone, sulfolane, and methylisopropylsulfone are more preferred. Acetonitrile, valeronitrile, hexanenitrile, 3-methoxypropionitrile, methoxyacetonitrile, diethylene glycol dimethyl ether, triethylene glycol dimethyl ether, 1,2-dimethoxyethane, sulfolane, and methylisopropylsulfone are particularly preferred.

Further, a compound that is liquid at ordinary temperature is preferred as an ionic liquid which can be used in

combination. Preferred examples of the ionic liquid include compounds obtained by combining cations, such as an imidazole cation, a pyrrolidinium cation, a pyridinium cation, a pyrrolium cation, a pyrazolium cation, and a phosphonium cation, with anions, such as a fluoride ion, a chloride ion, a bromide ion, an iodide ion, a perchlorate ion, a hydroxide ion, a methylsulfate ion, a toluenesulfonate anion, a tetrafluoroborate anion, a tetracyanoborate anion, a hexafluorophosphonate anion, a tetracyanoborate anion, a dicyanoimide anion, a trifluoromethanesulfonate anion, a bis(trifluoromethanesulfonyl)imide anion, a bis(pentafluoroethanesulfonyl)imide anion, and an (N-trifluoromethanesulfonyl-N-pentafluoroethanesulfonyl)imide anion. These solvents may be used singly or in combination of two or more. When two or more solvents are used in combination, the ratio can be arbitrarily selected.

Further, the electrolyte solution used in the dye-sensitized solar cell of the present invention may optionally contain a nitrogen-containing compound and other additives. The nitrogen-containing compound and other additives which can be used are not particularly limited, and the amount thereof to be added may be suitably selected depending on the purpose. It is preferred to add additives which have an effect of improving transport efficiency of the redox couple in an electrolyte solution, an effect of facilitating injection of a charge from a dye to an oxide semiconductor, an effect of preventing reverse electron transfer from an oxide semiconductor, and the like, and further increase the efficiency of a dye-sensitized solar cell or improve the stability of an electrolyte solution to thereby increase the durability of a dye-sensitized solar cell.

A sealing agent of the dye-sensitized solar cell of the present invention is used for laminating the first and the second conductive supports and sealing the electrolyte solution used in the charge transfer layer. The sealing agent is not particularly limited as long as the above purpose is achieved. Specific examples of the sealing agent can include an epoxy resin sealing agent, an acrylate resin sealing agent, a silicone resin sealing agent, a polyisobutylene resin sealing agent, an ionomer resin sealing agent, and a (modified) olefin resin sealing agent. Among these sealing agents, an epoxy resin sealing agent having high adhesive strength and excellent in solvent resistance and iodine resistance is preferably used.

Any epoxy resin sealing agents of a heat-curable type, an ultraviolet ray-curable type, and a heat and photo-curable type (or a type requiring both heat and light for curing) can be used as an epoxy resin sealing agent. Preferred is a sealing agent that can be applied to a screen printing method and a dispensing method and is excellent in properties after curing such as adhesive properties, heat resistance, moisture resistance, solvent resistance, light resistance, and gas barrier properties. An epoxy resin contained in the epoxy resin sealing agent is not particularly limited as long as it has at least two or more epoxy groups in one molecule. Examples of the epoxy resin include a Novolak type epoxy resin, a bisphenol type epoxy resin, a biphenyl type epoxy resin, a triphenylmethane type epoxy resin, a hydantoin type epoxy resin, an isocyanurate type epoxy resin, an aliphatic chain epoxy resin, a diglycidyl etherified product of difunctional phenols, a diglycidyl etherified product of difunctional alcohols, other glycidyl ether type epoxy resins, a glycidyl ester type epoxy resin, a glycidyl amine type epoxy resin, a cycloaliphatic epoxy resin, and halides and hydrogenated products thereof. Among them, preferred are solid or liquid epoxy resins such as glycidyl etherified epoxy resins, glycidyl aminated epoxy resins, glycidyl esterified epoxy resins, and halides and hydrogenated products thereof derived from polycondensates and modified products thereof, the polycondensates being obtained by allowing phenol novolac, cresol novolac, bisphenol A type novolac, trisphenol methane novolac, bisphenol A, bisphenol F, bisphenol S, fluorine bisphenol, tetrabromobisphenol A, terpenediphenol, 4,4'-biphenol, 2,2'-biphenol, 3,3',5,5'-tetramethyl-[1,1'-biphenyl]-4,4'-diol, hydroquinone, resorcin, naphthalenediol, tris-(4-hydroxyphenyl)methane, 1,1,2,2-tetrakis(4-hydroxyphenyl)ethane, or phenols (such as phenol, alkyl-substituted phenol, naphthol, alkyl-substituted naphthol, dihydroxybenzene, dihydroxynaphthalene) to react with formaldehyde, acetaldehyde, benzaldehyde, p-hydroxybenzaldehyde, o-hydroxybenzaldehyde, p-hydroxyacetophenone, o-hydroxyacetophenone, dicyclopentadiene, furfural, 4,4'-bis(chloromethyl)-1,1'-biphenyl, 4,4'-bis(methoxymethyl)-1,1'-biphenyl, 1,4-bis(chloromethyl)benzene, or 1,4-bis(methoxymethyl)benzene. More preferred epoxy resins are glycidyl etherified epoxy resins of phenol novolac, cresol novolac, trisphenol methane novolac, bisphenol A type novolac, bisphenol A, bisphenol F, bisphenol S, resorcin, and fluorene bisphenol, and halides and hydrogenated products thereof. Particularly preferred epoxy resins are phenol novolac type epoxy resins, cresol novolac type epoxy resins, trisphenol methane novolac type epoxy resins, bisphenol A type epoxy resins, bisphenol F type epoxy resins, bisphenol S type epoxy resins, and resorcin glycidyl ether, and halides and hydrogenated products thereof, Trisphenol methane novolac type epoxy resins and bisphenol A type epoxy resins are most preferred. These may be used singly or in combination. The epoxy resins as described above are useful for controlling the viscosity of a sealing agent. The sealing agent using these epoxy resins achieves the stacking operation of substrates at ordinary temperature during the preparation of the dye-sensitized solar cell of the present invention and facilitates the formation of a gap.

The composition of the epoxy resin sealing agents is not particularly limited, but it is common that, for example, the heat-curable type sealing agent contains an epoxy resin and a heat curing agent; the ultraviolet-curable type sealing agent contains an epoxy resin and a photopolymerization initiator; and the heat and photo-curable type sealing agent contains an epoxy resin, a heat curing agent, and a photoreaction initiator. All the compositions may contain further additives. For example, the heat-curable type sealing agent may optionally contain other heat-curable resins, a reaction accelerator, a filler, a coupling agent, a solvent, a stress relaxation agent, a viscosity controlling agent, a pigment, a

leveling agent, a defoaming agent, a spacer, and the like. The ultraviolet-curable type sealing agent may optionally contain other ultraviolet-curable resins, a photosensitizer, an ion catcher, a filler, a coupling agent, a solvent, a stress relaxation agent, a viscosity controlling agent, a pigment, a leveling agent, a defoaming agent, a spacer, and the like. The heat and photo-curable type sealing agent may optionally contain other heat-curable resins, other ultraviolet-curable resins, a reaction accelerator, a photosensitizer, an ion catcher, a filler, a coupling agent, a solvent, a stress relaxation agent, a viscosity controlling agent, a pigment, a leveling agent, a defoaming agent, a spacer, and the like. Among these sealing agents, a heat-curable type epoxy resin sealing agent is preferably used. More preferred is a heat-curable type epoxy resin sealing agent containing, as a heat curing agent, phenols, polyphenols, bisphenols, novolacs, amines, guanamines, imidazoles, hydrazides, or acid anhydrides. Among them, a heat-curable type epoxy resin sealing agent containing novolacs or hydrazides is particularly preferred. A heat-curable type epoxy resin sealing agent containing phenol novolacs, aromatic hydrazides, or aliphatic hydrazides having 6 or more carbon atoms is most preferred. These heat curing agents may be used singly or in combination. The epoxy resin sealing agents as described above are excellent in adhesive properties, moisture resistance, solvent resistance, and the like. Therefore, these epoxy resin sealing agents can particularly improve the durability of the dye-sensitized solar cell of the present invention.

Specific examples of the epoxy resin sealing agents include sealing agents described in JP 2002-368236 A, WO 2004/075333, WO 2007/046499, WO 2007/007671, and PCT/JP 2011/061166 (WO 2011/145551). Among them, sealing agents described in JP 2002-368236 A and PCT/JP 2011/061166 (WO 2011/145551) are particularly preferred.

Next, a general method of producing the dye-sensitized solar cell of the present invention will be described. First, a thin film of oxide semiconductor particulates (a semiconductor-containing layer) is prepared on the conductive support as described above. The thin film of oxide semiconductor particulates can be produced by a method of directly coating the conductive support by spraying or the like with oxide semiconductor particulates to form a thin film of semiconductor particulates; a method of electrically precipitating semiconductor particulates into the shape of a thin film using the conductive support as an electrode; a method of coating the conductive support with a slurry of semiconductor particulates or a paste containing particulates obtained by hydrolyzing a precursor of semiconductor particulates such as a semiconductor alkoxide, followed by drying and curing or firing the coating; or the like. The method of using a slurry is preferred in terms of the performance of an electrode using an oxide semiconductor. In the case of this method, the slurry is obtained by dispersing secondarily aggregated oxide semiconductor particulates in a dispersion medium by a conventional method so that the particulates have an average primary particle size of 1 to 200 nm.

The dispersion medium for dispersing a slurry is not particularly limited as long as it can disperse semiconductor particulates. Examples of the dispersion medium which can be used include water, alcohols such as ethanol, ketones such as acetone and acetylacetone, and hydrocarbons such as hexane. These may be mixed and used. Further, it is preferred to use water in terms of reducing the viscosity change of a slurry. Further, a dispersion stabilizer can be used in combination for the purpose of stabilizing the dispersion state of oxide semiconductor particulates. Examples of the dispersion stabilizer which can be used include acids such as acetic acid, hydrochloric acid, and nitric acid, and organic solvents such as acetylacetone, acrylic acid, polyethylene glycol, and polyvinyl alcohol.

The conductive support coated with a slurry may be fired. The firing temperature is generally 100°C or more, preferably 200°C or more. In addition, the upper limit is approximately equal to or lower than the melting point (softening point) of a support material, and is generally 900°C or less, preferably 600°C or less. Further, firing time is preferably, but not particularly limited to, about 4 hours or less. The thickness of the thin film on the conductive support is generally 1 to 200 $\mu$m, preferably 1 to 50 $\mu$m.

The thin film of oxide semiconductor particulates may be subjected to secondary treatment. The performance of the thin film of semiconductor particulates can also be improved, for example, by directly immersing the thin film together with the conductive support in a solution of an alkoxide, a chloride, a nitride, or a sulfide of the same metal as the semiconductor, followed by drying or refiring. Examples of the metal alkoxide include titanium ethoxide, titanium isopropoxide, titanium t-butoxide, and n-dibutyl-diacetyltin. In this case, an alcoholic solution is preferably used. Examples of the chloride include titanium tetrachloride, tin tetrachloride, and zinc chloride. In this case, an aqueous solution is preferably used. The oxide semiconductor thin film obtained in this way is composed of oxide semiconductor particulates.

Next, the sensitizing dye is adsorbed on the oxide semiconductor thin film. Examples of the method of adsorbing the sensitizing dye include a method of immersing the above conductive support provided with the semiconductor-containing layer in a solution in which a dye is dissolved in a solvent or a dispersion in which a dye is dispersed in a solvent. The concentration of the dye in the solution or the dispersion may be suitably determined depending on the type or the solubility of the dye. The immersion temperature is generally from ordinary temperature to the boiling point of the solvent. Further, the immersion time is generally from about 1 hour to 72 hours. Specific examples of the solvent which can be used for dissolving or dispersing a sensitizing dye include methanol, ethanol, acetonitrile, acetone, dimethylsulfoxide, dimethylformamide, n-propanol, i-propanol, t-butanol, and tetrahydrofuran. These solvents may be used singly or in combination of two or more in an arbitrary ratio. The concentration of the sensitizing dye in the solution or the dispersion is generally I $\times 10^{-6}$ to 1 M, preferably $1 \times 10^{-5}$ to $1 \times 10^{-1}$ M. By immersing the conductive support provided with the semiconductor-containing layer in the solution or the dispersion of the sensitizing dye in this way, a

conductive support having a dye-sensitized semiconductor-containing layer is obtained.

When dyes are mixed and used, the percentage of each dye is not particularly limited, but it is generally preferred to use each dye in an amount of at least about 10 mol%. When two or more dyes are carried in the semiconductor-containing layer using a solution in which the two or more dyes are dissolved or dispersed, the total concentration of the dyes in the solution may be the same as the concentration of a dye in the case where only one dye is carried in the layer. Further, the solvent used for each dye may be the same or different.

In order to prevent the association of dyes, it is effective that the dyes are carried in the semiconductor-containing layer in the presence of an inclusion compound. Examples of the inclusion compound used here include steroid compounds such as cholic acids, crown ether, cyclodextrin, calyx allene, and polyethylene oxide. It is preferred to use cholic acids. Among the cholic acids, it is preferred to use cholic acid, deoxycholic acid, chenodexycholic acid, methyl cholate, sodium cholate, ursodeoxycholic acid, and lithocholic acid. It is more preferred to use deoxycholic acid, chenodexycholic acid, ursodeoxycholic acid, and lithocholic acid. These inclusion compounds may be added to the dye solution or may be previously dissolved in a solvent before the dyes are dissolved or dispersed in the solvent. These inclusion compounds may also be used in combination. In this case, the ratio of the plurality of inclusion compounds can be arbitrarily selected. Further, after the dyes are carried in the semiconductor-containing layer, the layer may be treated with an amine compound such as 4-t-butylpyridine, pyridine, 4-methylpyridine, and triethylamine and an acid such as formic acid, acetic acid, and propionic acid. Examples of the treatment method to be employed include a method of immersing the conductive support provided with the semiconductor-containing layer in which the sensitizing dye is carried in an ethanol solution to which an amine compound or an acid is added; and a method of directly bringing an amine compound or an acid into contact with the conductive support provided with the semiconductor-containing layer in which the sensitizing dye is carried and washing the resulting conductive support with an organic solvent or water after a certain time, followed by drying.

Next, there will be described an example of a method of bonding together the conductive support (first conductive support) having a dye-sensitized semiconductor-containing layer and the conductive support (second conductive support) having a counter electrode, each obtained as described above, by using a sealing agent. First, a sealing agent, to which a spacer (gap controlling material) is added, is applied to the peripheral part of the conductive surface of any one of the conductive supports, into the shape of a darn leaving an inlet of a charge transfer layer, by using a dispenser, a screen printer, an ink jet printing machine, or the like. Subsequently, when the sealing agent contains a solvent, the sealing agent is heated, for example, with a hot-air dryer or the like to evaporate the solvent. Next, the other conductive support is stacked so that the conductive surfaces of the first and the second conductive supports may face each other, and the sealing agent is cured by heating and/or ultraviolet irradiation. Examples of the spacer used here include glass fiber, silica beads, polymer beads, and metal-coated particulates such as gold pearl and silver pearl. The diameter of these spacers is different depending on the purpose, but it is generally 1 to 100 μm, preferably 10 to 40 μm. The amount of the spacer used is generally 0.1 to 10 parts by mass, preferably 0.5 to 5 parts by mass, further preferably 1 to 2.5 parts by mass based on 100 parts by mass of the sealing agent The conditions of the heat cure of the sealing agent are generally 1 to 3 hours at 90 to 180°C Note that examples of the method of heat cure which can be employed include a method of performing heat cure by sandwiching the sealing agent with a heat pressing machine having two heating plates and a method of performing heat cure in an oven after fixing the sealing agent with a jig. Further, irradiation conditions of ultraviolet rays when an ultraviolet-curable type and a heat and photo-curable type sealing agent are used may be selected depending on the cure rate of the sealing agents. The gap between the first and the second conductive supports is generally 1 to 100 μm, preferably 4 to 50 μm.

The dye-sensitized solar cell of the present invention can be obtained by injecting a charge transfer layer into a gap between a pair of conductive supports bonded together as described above and then sealing the inlet of the charge transfer layer. An isobutylene resin, an epoxy resin, an UV-curable acrylic resin, and the like can be used as a sealant (sealing material) for sealing the inlet of a charge transfer layer. Any material other than that described above can be used as a sealant as long as the material has an effect of preventing the leak of the charge transfer layer from the inlet. A commercially available sealant can be used as a sealant. The UV-curable acrylic resin is particularly preferred.

On the other hand, a method described in WO 2007/046499 can also be employed as another method of producing a dye-sensitized solar cell. In this method, a dam of a sealing agent is provided in the peripheral part of the conductive surface of any one of the conductive supports without providing the inlet of a charge transfer layer; next, the same charge transfer layer as described above is arranged inside the dam of a sealing agent; the other conductive support is mounted and bonded together under a reduced pressure so that the conductive surfaces of the first and the second conductive supports may face each other, and a gap is formed at the same time; and then the sealing agent can be cured to obtain a dye-sensitized solar cell.

Fig. 1 is a schematic sectional view illustrating a major portion of the structure of the dye-sensitized solar cell of the present invention. In Fig. 1, reference numeral 1 represents a first conductive support in which the inside thereof has conductivity; reference numeral 2 represents a dye-sensitized semiconductor-containing layer; and reference numerals 1 and 2 are collectively called an oxide semiconductor electrode. Reference numeral 3 represents a second conductive support having a counter electrode in which platinum or the like is arranged on the conductive surface inside

the conductive support; reference numeral 4 represents a charge transfer layer arranged in the gap between the pair of conductive supports; reference numeral 5 represents a sealing agent; and reference numeral 6 represents a glass substrate.

## Examples

The present invention will be described in further detail below with reference to Examples, but the present invention is not limited to these Examples.

### Electrolyte Solution Preparation Example 1

An electrolyte solution 1 for dye-sensitized solar cells was obtained by dissolving each component in 3-methoxypropionitrile followed by mixing so as to obtain a concentration of 0.1 M of iodine, 0.1 M of lithium iodide (LiI) and 1.2 M of 1-methyl-3-propylimidazolium iodide as iodide, and 0.1 M of butyrylthiocholine iodide, which is a compound having, in a molecule thereof, both a thioester bond and a positively charged nitrogen atom, as an additive.

### Electrolyte Solution Preparation Examples 2 to 15 and 17 to 21

Electrolyte solutions 2 to 15 and 17 to 21 for dye-sensitized solar cells were obtained in the same manner as in Electrolyte Solution Preparation Example 1 except that the additive was changed to each compound shown in Table 1.

### Electrolyte Solution Preparation Example 16

An electrolyte solution 16 for dye-sensitized solar cells was obtained in the same manner as in Electrolyte Solution Preparation Example 1 except that butyrylthiocholine iodide which is an additive was not used.

### Evaluation Test 1 (Heat-resistant Stability Evaluation of Electrolyte Solution)

Electrolyte solutions 1 to 21 obtained in Electrolyte Solution Preparation Examples 1 to 21 were each put in a brown sample bottle in an amount of 1 mL and heated in a sealed state in a dryer at 85°C for 20 hours. Subsequently, the state of the electrolyte solutions was visually observed. When no change was observed in the state, the electrolyte solution was rated as "O"; when brown color of iodine bleached or when a precipitate produced in a solution, the electrolyte solution was rated as "X". The results are shown in Table 1

### Evaluation Test 2 (Heat-resistant Stability Evaluation of Platinum against Electrolyte Solution)

Platinum was vapor-deposited to a thickness of 50Å by sputtering on the conductive surfaces of FTO conductive glass supports which are conductive supports, and the resulting supports with platinum were cut into a size of 1 cm x 2 cm to obtain test pieces. One milliliter of each of the electrolyte solutions 1 to 21 and one of the above test pieces were put in a brown sample bottle and heated in a sealed state in a dryer at 85°C for 20 hours. Subsequently, the test pieces were removed, and the state of platinum was visually observed. When no change was observed in the state of platinum, the electrolyte solution was rated as "O"; when black color of platinum bleached, the electrolyte solution was rated as "X". The results are shown in Table 1.

Table 1

| Electrolyte solution number | Additive | Additive structure | Evaluation test 1 | Evaluation test 2 |
|---|---|---|---|---|
| 1 | Bytyrylthiocholine iodide | Formula (4) | ○ | ○ |
| 2 | Acetylthiocholine iodide | Formula (5) | ○ | ○ |
| 3 | Benzoylthiocholine iodide | Formula (6) | ○ | ○ |
| 4 | S-phenylthioacetic acid | Formula (7) | × | ○ |

(continued)

| Electrolyte solution number | Additive | Additive structure | Evaluation test 1 | Evaluation test 2 |
|---|---|---|---|---|
| 5 | S-methyl furancarbothiate | Formula (8) | ○ | ○ |
| 6 | 2,4-Thiazolidinedione | Formula (9) | × | ○ |
| 7 | Thioacetamide | Formula (10) | ○ | ○ |
| 8 | N-methylpyrrolidine-2-thione | Formula (11) | ○ | ○ |
| 9 | Guanidine thiocyanate | Formula (12) | ○ | ○ |
| 10 | Methylisothiocyanate | Formula (13) | ○ | ○ |
| 11 | DMSO | Formula (14) | ○ | × |
| 12 | Sodium sulfate | $Na_2SO_4$ | ○ | × |
| 13 | Sodium thiosulfate | $NaS_2O_3$ | × | ○ |
| 14 | Sodium thiocyanate | NaSCN | ○ | ○ |
| 15 | Potulium thiocyanate | KSCN | ○ | ○ |
| 16 | Nothing | - | ○ | × |
| 17 | 1-Ethyl-3-methylimidazolium thiocyanate | Formula (15) | ○ | ○ |
| 18 | Cyclohexylisothiocyanate | Formula (16) | ○ | ○ |
| 19 | Ethyltrimethylammonium iodide | Formula (17) | ○ | × |
| 20 | Acetylcholine iodide | Formula (18) | ○ | × |
| 21 | Butyrylcholine iodide | Formula (19) | ○ | × |

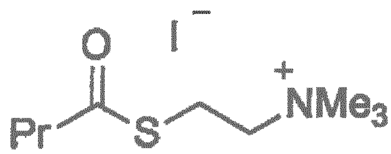 (4) 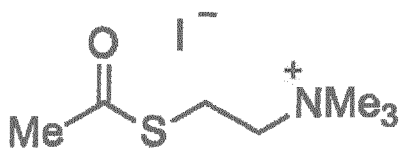 (5)

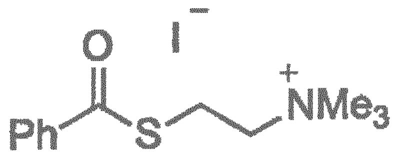 (6) 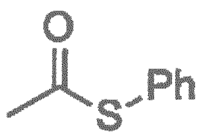 (7) 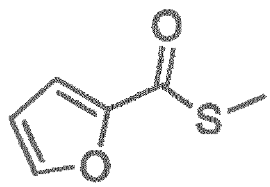 (8)

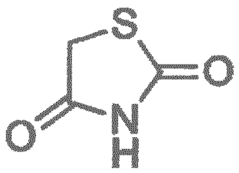 (9) 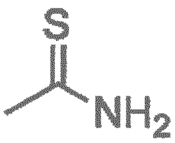 (10) 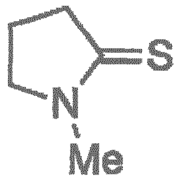 (11)

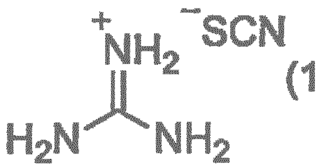 (12) 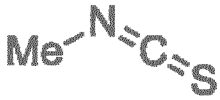 (13) 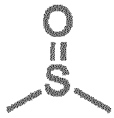 (14)

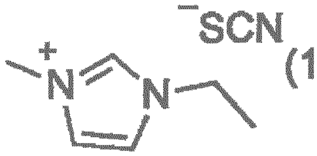 (15) 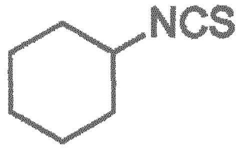 (16) 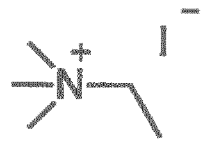 (17)

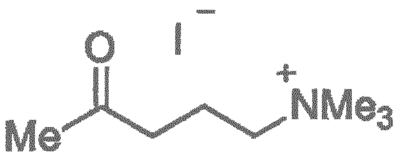 (18) 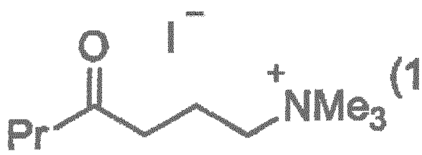 (19)

Example 1

A paste of $TiO_2$ particulates (having an average particle size of 20 nm) in terpineol was applied to the conductive surface of an FTO conductive glass support which is a conductive support with a screen printer and fired at 450°C for 30 minutes to prepare a conductive support having a semiconductor-containing layer (having a thickness of 10 μm, a minor axis width of 5 mm, and a major axis width of 4 cm). The resulting conductive support provided with the semiconductor-containing layer was immersed in a dye solution, which was obtained by dissolving a dye described in Example 6 of WO 2007/100033 (a dye represented by the following formula (3)) in acetone so as to obtain a concentration of 1.6 $\times 10^{-4}$ M of the dye, at room temperature for 24 hours to prepare an oxide semiconductor electrode. Next, Pt was vapor-deposited to a thickness of 50Å on the conductive surface of another FTO conductive glass support to prepare a counter electrode. A sealing agent, which was prepared by adding 2.5 mass% of gold pearl (having a pearl diameter of 20 μm) as a spacer to an epoxy resin sealing agent described in Sealing Agent Preparation Example 3 of WO 2011/14551, was applied to the peripheral edge part of the resulting counter electrode using a screen printer so that an inlet of a charge transfer layer might be left, and the solvent was removed by heating at 90°C for 18 minutes with a hot-air dryer. Subsequently, the oxide semiconductor electrode described above was stacked on the sealing agent so that the conductive surface of the counter electrode and the semiconductor-containing layer faced each other, and the sealing agent was cured at 150°C for 60 minutes under a pressure of 2.5 kg/$cm^2$ using a heat pressing machine, thereby obtaining a cell in which both conductive supports are bonded together. The resulting cell was filled with the electrolyte solution 1 obtained in the Electrolyte Solution Preparation Example from the inlet of the cell, and then the inlet was sealed with an UV-curable acrylic resin, thereby obtaining a dye-sensitized solar cell of the present invention (cell 1).

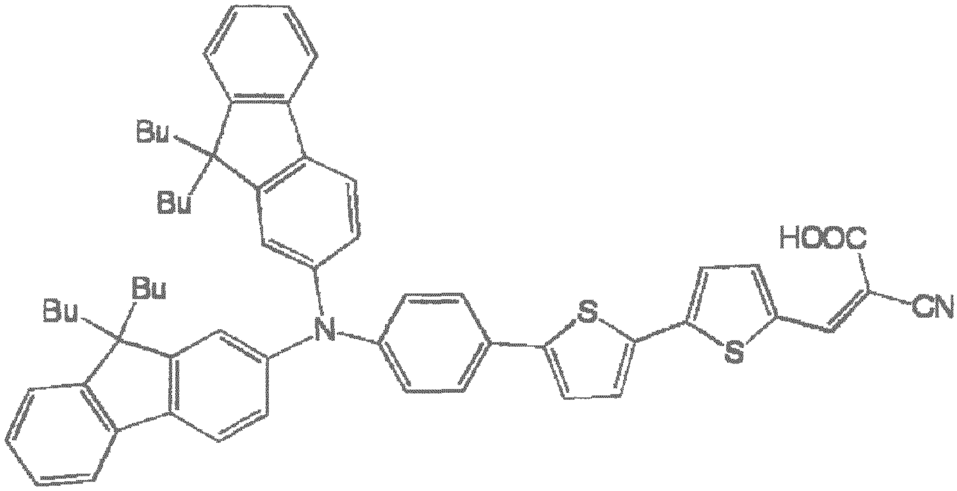

(3)

Example 2

A dye-sensitized solar cell of the present invention (cell 2) was obtained in the same manner as in Example 1 except that the electrolyte solution 1 was changed to the electrolyte solution 2 obtained in the Electrolyte Solution Preparation Example.

Comparative Examples 1 to 13

Dye-sensitized solar cells for comparison (cells 3 to 15) were obtained in the same manner as in Example 1 except that the electrolyte solution 1 was changed to the electrolyte solutions 5, 7 to 10, and 14 to 21 obtained in the Electrolyte Solution Preparation Examples, respectively.

Evaluation Test 3 (Measurement of Initial Photoelectric Conversion Efficiency (Initial Eff))

The cells 1 and 2 obtained in Examples 1 and 2, respectively, and the cells 3 to 15 obtained in Comparative Examples 1 to 13, respectively were measured for photoelectric conversion ability. The photoelectric conversion efficiency (Eft) calculated from open-circuit voltage, short-circuit current, and a shape factor was measured with a solar simulator (WXS-155S-10, manufactured by WACOM ELECTRIC CO., LTD.), in which a 1-kW xenon lamp (manufactured by WACOM ELECTRIC CO., LTD.) was used as a light source, and 100 mW/cm$^2$ was obtained through an AM 1.5 filter. The results are shown in Table 2.

Evaluation Test 4 (Accelerated Heat Resistance Test)

The cells 1 and 2 obtained in Examples 1 and 2, respectively, and the cells 3 to 15 obtained in Comparative Examples 1 to 13, respectively, were subjected to accelerated heat resistance test at 85°C. Each cell was put in an aluminum bag, held at 85°C for 500 hours, and then measured for the photoelectric conversion efficiency (Eff) in accordance with the test method of the Evaluation Test 3. Further, the Eff degradation rate was calculated by the following Formula. The results are shown in Table 2.

$$\text{Eff degradation rate (\%)} = 100 \times [(\text{Initial Eff} - \text{Eff after 500 hours at 85°C})/(\text{Initial Eff})]$$

Table 2

| | Cell number | Electrolyte solution number | Initial Eff | Eff after 500hr at 85°C | Eff degradation rate |
|---|---|---|---|---|---|
| Example 1 | 1 | 1 | 5.6 | 5.3 | 5% |
| Example 2 | 2 | 2 | 5.4 | 5.1 | 6% |

(continued)

| | Cell number | Electrolyte solution number | Initial Eff | Eff after 500hr at 85°C | Eff degradation rate |
|---|---|---|---|---|---|
| Comparative Example 1 | 3 | 5 | 5.2 | 0.9 | 83% |
| Comparative Example 2 | 4 | 7 | 5.2 | 4.2 | 19% |
| Comparative Example 3 | 5 | 8 | 5.2 | 2.4 | 54% |
| Comparative Example 4 | 6 | 9 | 5.6 | 3.6 | 36% |
| Comparative Example 5 | 7 | 10 | 5.1 | 4.2 | 18% |
| Comparative Example 6 | 8 | 14 | 5.4 | 4.2 | 22% |
| Comparative Example 7 | 9 | 15 | 5.0 | 3.5 | 30% |
| Comparative Example 8 | 10 | 16 | 5.1 | 0.7 | 86% |
| Comparative Example 9 | 11 | 17 | 4.5 | 2.5 | 44% |
| Comparative | 12 | 18 | 2.5 | 1.2 | 52% |
| Comparative Example 11 | 13 | 19 | 5.6 | <0.1 | >99% |
| Comparative Example 12 | 14 | 20 | 5.6 | <0.1 | >99% |
| Comparative Example 13 | 15 | 21 | 5.5 | <0.1 | >99% |

In the Evaluation Test 1 in which the stability of an electrolyte solution was tested, the electrolyte solutions each containing a compound having, in a molecule thereof, both a thioester bond and a positively charged nitrogen atom typified by the electrolyte solutions 1 to 3 had satisfactory storage stability. On the other hand, systems having extremely poor stability were also present such as the electrolyte solutions 4, 6, and 13 because although these electrolyte solutions each contain a similar compound, an oxidation-reduction reaction between a sulfur atom and iodine has proceeded. Further, in the Evaluation Test 2 in which the stability of platinum of the counter electrode was tested, the electrolyte solutions each containing a compound having, in a molecule thereof, both a thioester bond and a positively charged nitrogen atom typified by the electrolyte solutions 1 to 3 had satisfactory stability of platinum. On the other hand, in the electrolyte solutions 11, 12, 16, and 19 to 21, platinum was corroded by iodine in the electrolyte solutions. From these results, it is apparent that the electrolyte solutions each containing a compound having, in a molecule thereof, both a thioester bond and a positively charged nitrogen atom are excellent in both the stability of the electrolyte solutions and the stability of platinum which is a counter electrode material.

Next, the cells 1 and 2 of the present invention were prepared using the electrolyte solutions 1 and 2, respectively, which provided satisfactory results in the Evaluation Tests 1 and 2, and the cells 3 to 15 having the same configuration were prepared using the electrolyte solutions 5, 7, 8,9,10, 14, 15, 16, 17, 18, 19, 20, and 21, respectively. Then, these cells were subjected to the initial Eff measurement and the accelerated heat resistance test at 85°C for 500 hours. As a result, both the initial Eff and the Eff after being held at 85°C for 500 hours of the cells 1 and 2 of the present invention using the electrolyte solutions 1 and 2, respectively, were satisfactory, and the Eff degradation rate of these cells was also about 5% On the other hand, the cell 10 using the electrolyte solution 16, which did not contain a sulfur additive, had an extremely poor degradation rate of 86%, and the cell 3 using the electrolyte solution 5, to which a compound having only a thioester bond was added, also had a degradation rate of 83%. Further, the cells 4 and 5 using the electrolyte solutions 7 and 8, respectively, to which a thioamide compound was added, and the cells 6 to 9, 11, and 12

using the electrolyte solutions 9, 10, 14, 15, 17, and 18, respectively, to which a compound containing thiocyanate was added, had a degradation rate of 18% or more. Thus, all the cells had poor durability. Further, all the cells 13 to 15 using the electrolyte solutions 19, 20, and 21, respectively, to which a compound having, in a molecule thereof, no thioester bond and only a positively charged nitrogen atom was added, had a degradation rate of 99% or more, resulting in extremely poor cell durability. Note that it has been proved that the same desired effect as described above can be obtained also by a dye-sensitized solar cell prepared using a known non-ruthenium dye other than the compound represented by formula (3).

It is apparent from the above results that the dye-sensitized solar cell of the present invention using an electrolyte solution containing a compound having, in a molecule thereof, both a thioester bond and a positively charged nitrogen atom has excellent photoelectric conversion efficiency and heat-resistant durability

Industrial Applicability

The dye-sensitized solar cell of the present invention in which a sensitizing dye is an organic non-ruthenium dye; a counter electrode contains platinum; and a charge transfer layer comprises an electrolyte solution containing iodine, iodide ions, and a compound having, in a molecule thereof, both a thioester bond and a positively charged nitrogen atom has excellent conversion efficiency and high durability. Therefore, a dye-sensitized solar cell which is hardly degraded even if used for a long period of time can be provided using a non-ruthenium dye which has few restrictions on resources and is wide in the width of molecular design.

Reference Signs List

1 Conductive support
2 Dye-sensitized semiconductor-containing layer
3 Counter electrode
4 Charge transfer layer
5 Sealing agent
6 Glass substrate

**Claims**

**1.** A dye-sensitized solar cell comprising: a first conductive support having a dye-sensitized semiconductor-containing layer; a second conductive support having a counter electrode provided in a position where the semiconductor-containing layer and the counter electrode are opposite to each other at a predetermined interval; a charge transfer layer sandwiched in a gap between the first and the second conductive supports; and a sealing agent provided in a peripheral part of the first and the second conductive supports in order to seal the charge transfer layer, wherein the dye is an organic non-ruthenium dye; the counter electrode contains platinum; and the charge transfer layer comprises an electrolyte solution containing iodine, iodide ions, and a compound having, in a molecule thereof, both a thioester bond and a positively charged nitrogen atom.

**2.** The dye-sensitized solar cell according to claim 1, wherein the compound having, in a molecule thereof, both a thioester bond and a positively charged nitrogen atom has a structure represented by formula (1):

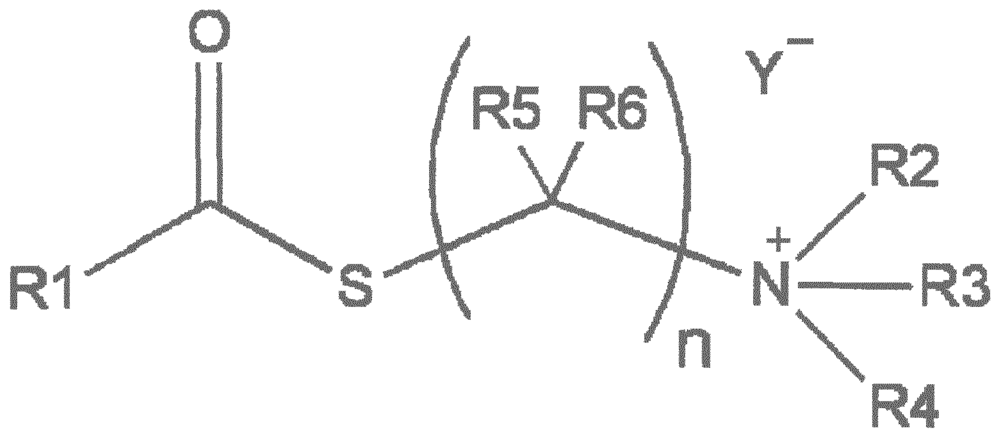

(1)

wherein R1, R2, R3, R4, R5, and R6 each independently represent an aliphatic hydrocarbon residue having 1 to 10 carbon atoms which may have one or more substituents selected from the group consisting of a halogen atom, an alkoxy group, an ester group, an acyl group, an amino group, an amide group, an alkyl group, an alkynyl group, an alkynyl group, an aryl group, a cyano group, an isocyano group, a nitro group, a nitroso group, a hydroxyl group, a phosphate group, a sulfinyl group, and a sulfinyl group, an aromatic hydrocarbon residue which may have one or more substituents selected from the group consisting of a halogen atom, an alkoxy group, an ester group, an acryl group, an amino group, an amide group, an alkyl group, an alkenyl group, an alkenyl group, an aryl group, a cyano group, an isocyano group, a nitro group, a nitroso group, a hydroxyl group, a phosphate group, a sulfinyl group, and a sulfonyl group, a heterocyclic residue which may have one or more substituents selected from the group consisting of a halogen atom, an alkoxy group, an ester group, an acyl group, an amino group, an amide group, an alkyl group, an alkenyl group, an alkynyl group, an aryl group, a cyano group, an isocyano group, a nitro group, a nitroso group, a hydroxyl group, a phosphate group, a sulfinyl group, and a sulfonyl group, or a hydrogen atom; any two selected from R1, R2, R3, R4, R5, and R6 may be combined to form a ring; n represents an integer of 1 to 6; and $Y^-$ represents a monovalent anion.

3. The dye-sensitized solar cell according to claim 2, wherein the compound represented by formula (1) is a compound having a thiocholine residue.

4. The dye-sensitized solar cell according to claim 2 or 3, wherein the compound represented by formula (1) is a compound having a halide ion.

5. The dye-sensitized solar cell according to claim 1, wherein the sealing agent is an epoxy resin sealing agent.

6. The dye-sensitized solar cell according to claim 1, wherein a semiconductor in the semiconductor-containing layer is titanium oxide in the form of particulates or a composite titanium oxide in the form of particulates.

7. The dye-sensitized solar cell according to claim 1, wherein the organic non-ruthenium dye has a structure represented by formula (2):

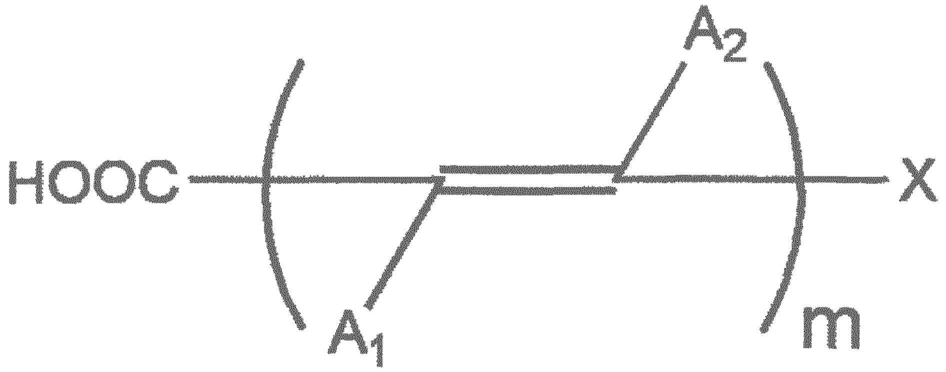

(2)

wherein $A_1$ and $A_2$ each independently represent a carboxyl group, a cyano group, an alkoxycarbonyl group, an acyl group, a nitro group, a cyclic hydrocarbon residue, a heterocyclic residue, an amino group, a hydroxyl group, a hydrogen atom, a halogen atom, or an alkyl group; X represents an aromatic hydrocarbon residue, a heterocyclic residue, or an amino group; m represents an integer of 1 to 6; when m is 2 or more and a plurality of $A_1$ and $A_2$ are present, each $A_1$ and each $A_2$ independently of each other represent said group, residue or atom which may be the same or different; and any two of $A_1$ or each $A_1$ when a plurality of $A_1$ are present, $A_2$ or each $A_2$ when a plurality of $A_2$ are present, and X may be combined to form a ring.

**8.** The dye-sensitized solar cell according to claim 7, wherein $A_1$ in formula (2) is a cyano group or a carboxyl group.

**9.** The dye-sensitized solar cell according to claim 7 or 8, wherein X in formula (2) is a (poly)ethenyl group or a (poly)thiophenyl group each having a triphenylamine derivative.

FIG.1

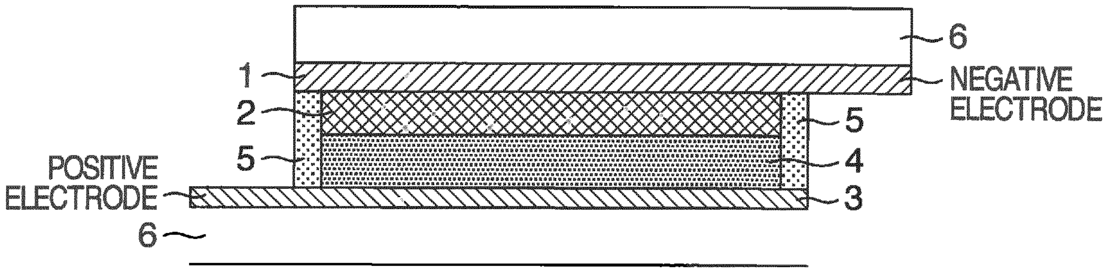

## INTERNATIONAL SEARCH REPORT

International application No. PCT/JP2013/082004

A. CLASSIFICATION OF SUBJECT MATTER
*H01M14/00*(2006.01)i, *C09B23/00*(2006.01)i, *H01B1/12*(2006.01)i, *H01L31/04*(2014.01)i, *H01M2/08*(2006.01)i

According to International Patent Classification (IPC) or to both national classification and IPC

B. FIELDS SEARCHED

Minimum documentation searched (classification system followed by classification symbols)
H01M14/00, C09B23/00, H01B1/12, H01L31/04, H01M2/08

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2013 |
| Kokai Jitsuyo Shinan Koho | 1971-2013 | Toroku Jitsuyo Shinan Koho | 1994-2013 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| A | WO 2012/014414 A1 (Shimane-Ken), 02 February 2012 (02.02.2012), entire text & US 2013/0125979 A1 & EP 2600464 A1 & CN 103038935 A & KR 10-2013-0040247 A | 1-9 |
| A | JP 2003-234486 A (Seiko Epson Corp.), 22 August 2003 (22.08.2003), entire text (Family: none) | 1-9 |
| A | JP 2009-199810 A (Konica Minolta Business Technologies, Inc.), 03 September 2009 (03.09.2009), entire text (Family: none) | 1-9 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 24 December, 2013 (24.12.13) | 14 January, 2014 (14.01.14) |

| Name and mailing address of the ISA/ | Authorized officer |
|---|---|
| Japanese Patent Office | |
| Facsimile No. | Telephone No. |

Form PCT/ISA/210 (second sheet) (July 2009)

INTERNATIONAL SEARCH REPORT

International application No.
PCT/JP2013/082004

C (Continuation). DOCUMENTS CONSIDERED TO BE RELEVANT

| Category* | Citation of document, with indication, where appropriate, of the relevant passages | Relevant to claim No. |
|---|---|---|
| P,A | WO 2013/061958 A1 (Nippon Kayaku Co., Ltd.), 02 May 2013 (02.05.2013), entire text (Family: none) | 1-9 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2012014414 A
- JP 2010192226 A
- JP 4264507 B
- WO 2009069757 A
- WO 2006080384 A
- WO 2002011213 A
- WO 2006126538 A
- JP 3731752 B
- JP 2002334729 A
- JP 2002512729 A
- JP 2003007358 A
- JP 2003017146 A
- JP 2003059547 A
- JP 2003086257 A
- JP 2003115333 A
- JP 2003132965 A
- JP 2003142172 A
- JP 2003151649 A
- JP 2003157915 A
- JP 2003282165 A
- JP 2004014175 A
- JP 2004022222 A
- JP 2004022387 A
- JP 2004227825 A
- JP 2005005026 A
- JP 2005019130 A
- JP 2005135656 A
- JP 2006079898 A
- JP 2006134649 A
- JP 2007 A
- JP 149570 A
- JP 2008021496 A
- JP 2010146864 A
- WO 2002001667 A
- WO 2002071530 A
- WO 2004082061 A
- WO 2006082061 A
- WO 2007100033 A
- WO 2009020098 A
- WO 2010021378 A
- JP 2007149570 A
- JP 2002368236 A
- WO 2004075333 A
- WO 2007046499 A
- WO 2007007671 A
- JP 2011061166 W
- WO 2011145551 A
- JP 2002 A
- JP 368236 A
- WO 201114551 A


### Non-patent literature cited in the description


- *Nature,* 1991, vol. 353, 737-740
- *Chemical Reviews,* 2010, vol. 110 (11), 6616-6631
- *The Journal of Physical Chemistry C,* 2009, vol. 113, 21779-21783